(19) Europäisches Patentamt
European Patent Office
Office européen des brevets

(11) Publication number: 0 387 068 A1

(12) **EUROPEAN PATENT APPLICATION**

(21) Application number: 90302497.4

(51) Int. Cl.[5]: C12P 41/00

(22) Date of filing: 08.03.90

(30) Priority: 08.03.89 US 320410

(43) Date of publication of application:
12.09.90 Bulletin 90/37

(84) Designated Contracting States:
AT BE CH DE DK ES FR GB GR IT LI LU NL SE

(71) Applicant: WISCONSIN ALUMNI RESEARCH FOUNDATION
614 North Walnut Street
Madison, WI 53705(US)

(72) Inventor: Sih, Charles J.
6322 Landfall Drive
Madison, Wisconsin 53705(US)

(74) Representative: Ellis-Jones, Patrick George Armine et al
J.A. KEMP & CO. 14 South Square Gray's Inn
London WC1R 5LX(GB)

# (54) Improving the enantioselectivity of biocatalytic resolution of racemic compounds.

(57) A method of improving the enantioselectivity of biocatalytic resolution of racemic compounds by using an inhibitor to selectively block one of the competing enantiomer reactions.

FIGURE 1

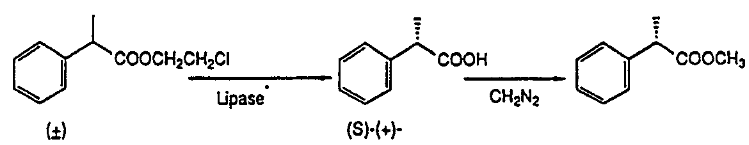


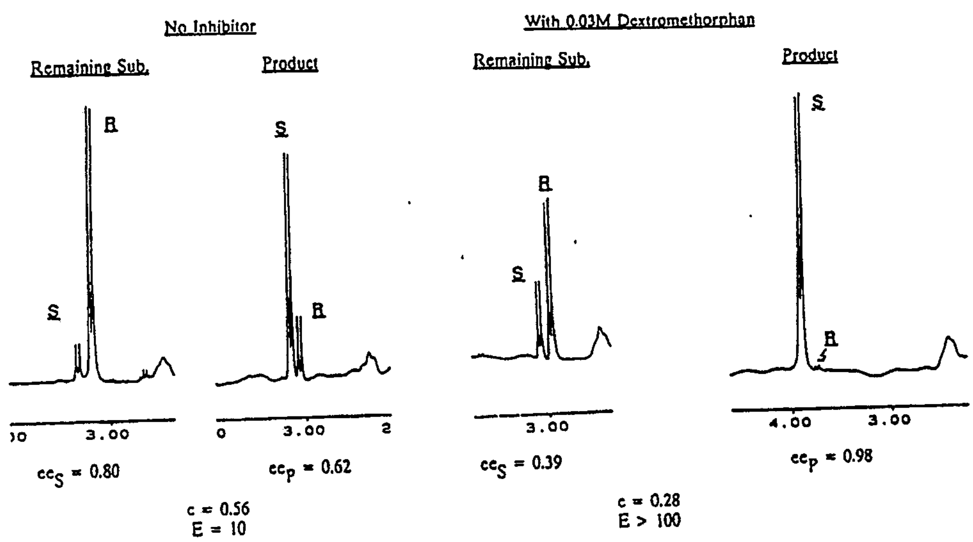


*Crude powder of *Candida cylindracea* (Sigma), 24°C.

EP 0 387 068 A1

# IMPROVING THE ENANTIOSELECTIVITY OF BIOCATALYTIC RESOLUTION OF RACEMIC COMPOUNDS

## Technical Field

The present invention relates to a new concept for improving the enantioselectivity of biocatalytic kinetic resolution of racemic compounds. More specifically, this invention is concerned with the use of inhibitors to selectively inhibit one of the competing biocatalytic reactions such as the one involving the slower-reacting enantiomer, thereby improving the enantioselectivity of the overall biocatalytic resolution process.

## Background and Summary of the Invention

Biocatalysts (enzymes, microorganisms, immobilized cells, etc.) have been widely used for the resolution of amino acids [G. Schmidt-Kastner and P. Egerer in Biotechnology, 6a, K. Kieslich (ed.), Verlag Chemie, 1984, p. 388], racemic alcohols [V. Krasnobajew, ibid., p. 98], and racemic acids [Sheldon et al in Stereoselectivity of Pesticides, E. J. Ariens, J. J. S. Van Rensen and W. Welling (eds.), Elsevier, Amsterdam, 1988, p. 409]. However, in many cases the enantioselectivity of the biocatalytic resolution process is low or moderate [C. J. Sih and S. H. Wu in Topics in Stereochemistry, 39, 1989], so that the biocatalytic system is unsuitable for the industrial separation of the racemates. Alternatively, one has to resort to the tedious task of screening a large number of biocatalysts before a suitable one is found.

Accordingly, the present invention provides a new method for enhancing the enantioselectivity of biocatalytic resolution processes to extend the usefulness of biocatalysts of low to moderate enantioselectivities. The laborious task of screening for a new suitable biocatalyst is reduced. The unique feature of this invention is the discovery that it is possible to selectively inhibit one of the competing reactions preferably the one involving the slower-reacting enantiomer.

## Brief Description of the Drawing

Figure 1 illustrates graphically the enhanced selectivity to the (S)-(+)- enantiomer of chloroethyl-2-phenylpropionate by the use of dextromethorphan as an inhibitor during hydrolysis.

## Detailed Description of the Invention

### 1. Principles of Biocatalytic Kinetic Resolution

A normal biocatalytic resolution process includes A and B which are the fast- and slow-reacting enantiomers that compete for the same site on the biocatalyst. For a simple three-step kinetic mechanism (1), assuming the reaction is virtually irreversible and there is no product inhibition,

$$\begin{array}{l} Enz + A \underset{k_2}{\overset{k_1}{\rightleftarrows}} EA \xrightarrow{k_3} EP \xrightarrow{k_5} E + P \\ Enz + B \underset{k_2'}{\overset{k_1'}{\rightleftarrows}} EB \xrightarrow{k_3'} EQ \xrightarrow{k_5'} E + Q \end{array} \qquad (1)$$

the ratio of the two partial reaction rates ($v_A$ and $v_B$) may be shown by steady-state kinetics to be

$$\frac{v_A}{v_B} = \frac{k_{cat_A}}{k_{cat_B}} \cdot \frac{K_B}{K_A} \cdot \frac{A}{B} \qquad (2)$$

where $K_{cat_A}$ , $K_A$ and $K_{cat_B}$ , $K_B$ denote catalytic constants and Michaelis constants of the fast- and slow-reacting enantiomers respectively.

Integration of equation 2 affords the homocompetitive equation 3 [C. S. Chen et al, J. Am. Chem. Soc., 102 (1982) 7294], which reveals that the discrimination between two competing enantiomers (A and B) by biocatalysts is dictated by E (the ratio of the specificity constants $k_{cat}/K$).

$$\frac{\ln[A/A_o]}{\ln[B/B_o]} = \frac{k_{cat_A}/K_A}{k_{cat_B}/K_B} = E \qquad (3)$$

The enantiomeric ratio, E, is an intrinsic property of that biocatalytic system and is an index of enantioselectivity. It is independent of enzyme and substrate concentration as well as the extent of conversion (c). This constant, E, allows the investigator to compare the enantioselectivity of different biocatalytic systems.

## 2. Experimental Determination of E

Equation 3 may be readily transformed into a more useful format to relate to variables: the extent of conversion (c); the enantiomeric excess of the recovered substrate fraction ($ee_S$); and the enantiomeric ratio (E) (eq. 4).

$$\frac{\ln[(1 - c)(1 - ee_S)]}{\ln[(1 - c)(1 + ee_S)]} = E \qquad (4)$$

where $c = 1 - [(A+B)/(A_o+B_o)]$, $ee_S = (B-A)/(A+B)$, and $A_o$ and $B_o$ denote initial concentrations. It is evident that knowledge of any two of the varibles allows the definition of the third.

Experimentally, if one determines the enantiomeric excess of the remaining substrate ($ee_S$) and the product fraction ($ee_P$), one can readily calculate c using the following relationship (eq. 5):

$$c = \frac{ee_S}{ee_S + ee_P} \qquad (5)$$

Substitution of the values for $ee_S$ and c into equation 4 allows the calculation of E [C. S. Chen et al, J. Am. Chem. Soc., 102 (1982) 7294].

## 3. Theory of Enantioselective Inhibition

In the following A and B are again the competing fast- and slow-reacting enantiomers. A is converted into the product, P, whereas B is converted into the product, Q, by the biocatalyst. I is a non-competitive inhibitor that forms complexes with the free enzyme, E, and with the enzyme-substrate complexes, EA and EB, to yield EI, EAI and EBI, respectively. All these complexes (EA, EAI, EB and EBI) are transformed into

products P and Q respectively. The following scheme summarizes the inhibition equilibria:

$$\begin{array}{ccccccccc} Q + E & \xleftarrow{k_q} & EB & \underset{K_B}{\overset{B}{\rightleftarrows}} & E & \underset{K_A}{\overset{A}{\rightleftarrows}} & EA & \xrightarrow{k_p} & E + P \\ & & I\downarrow\uparrow K_{iB} & & I\downarrow\uparrow K_i & & I\downarrow\uparrow K_{iS} & & \\ Q+EI & \xleftarrow{\alpha k_q} & EBI & \underset{B}{\overset{K_{B'}}{\rightleftarrows}} & EI & \underset{K_{A'}}{\overset{A}{\rightleftarrows}} & EAI & \xrightarrow{\beta k_p} & EI+P \end{array}$$

At any [I], the overall velocity at which P is formed is $k_p[EA] + \beta k_p[EAI]$, where $\beta < 1$ and the overall velocity at which Q is formed is $k_q[EB] + \alpha k_q[EBI]$ where $\alpha < 1$. It can be seen from the equilibria that an infinitely high substrate concentration [A] and [B] will not drive all the enzyme to the [EA] and [EB] forms. At any given inhibitor concentration, a portion of the enzyme will exist as the less productive [EAI] and [EBI] forms.

In the simplest case, where $K_i = K_{i_R} = K_{i_S}$ and $k_A = K_A'$ and $KB = K_B'$, it can be shown that the discrimination process is governed by equation 6.

$$\frac{\ln[A/A_O]}{\ln[B/B_O]} = E \cdot \frac{K_i + \beta I}{K_i + \alpha I} \qquad (6)$$

Hence, equation 6 differs from the classical homocompetitive equation 3 by the presence of an additional factor: $(K_i + \beta I)/(K_i + \alpha I)$. It is readily apparent that when $\beta > \alpha$, an enhancement of the E value results. When $\beta = 1$, there is no inhibition of P formation (ES→P = ESI→P). This mode of inhibition may be termed as partial non-competitive enantioselective inhibition. For a description of simple conventional partial non-competitive inhibition, see I. H. Segel [Enzyme Kinetics, Wiley-Interscience, N.Y., 1975, p. 166].

## 4. General Disclosure of the Invention

Broadly, this invention comprises the use of the principle of differential inhibition as a means of improving the enantioselectivity of biocatalytic systems for the preparation of optically-active compounds. To demonstrate the efficacy of this selective inhibition, the following two classes of compounds were selected as model substrates and the extracellular microbial lipases as the biocatalyst. However, this concept is general, and is applicable to all biocatalytic kinetic resolution processes. Thus, one need only to match a particularly desirable biocatalyst and inhibitor with the initial racemic substrate to be resolved.

More specifically, examples of racemic substrates to which the principles of the present invention are applicable include any chiral racemic esters such as esters of amino, hydroxy and halo acids and their derivatives. It is typically desirable to obtain one of the two optically active stereoisomeric forms of the above racemic compounds in accordance with the method of the present invention.

Turning now to the two model substrates previously noted above, 2-Arylpropionic acids contain a center of asymmetry and therefore, they exist in two stereoisomeric forms: the R and S forms. However, the antiinflammatory activities of the S-enantiomers of 2-arylpropionic acids are generally greater than their R-enantiomers [Non-Steroidal Antiinflammatory Drugs, J. G. Lombardino (ed.), John Wiley & Sons, N.Y., 1985, p. 303]. Similarly, the R form of 2-aroylpropionic acids possess the herbicidal activity whereas the S form is relatively inert [Stereoselectivity of Pesticides, E. J. Ariens, J. J. S. Van Rensen, and W. Welling (eds.), Elsevier, Amsterdam, 1988, p. 39]. The known chemical methods for resolving the two enantiomers are complicated, need costly optically-active reactants, e.g., the chiral agents such as methylbenzylamine, in more than stoichiometric amounts, and in many cases do not achieve satisfactry yields and thus are unsuitable for industrial use. Therefore, simple and inexpensive methods for the resolution of the enantiomers of these two classes of compounds are needed.

Accordingly, for the above two model substrates, the present invention consists in the use of a biocatalyst that is capable of hydrolyzing a racemic ester, and, by the addition of a chiral amine inhibitor to the reaction, the enantioselectivity is markedly improved. More specifically, using (±)-2-arylpropionic esters as the substrate, the lipase of Candida cylindracea as the biocatalyst and dextromethorphan as the chiral amine inhibitor, the enantioselectivity (or the E value) is markedly elevated.

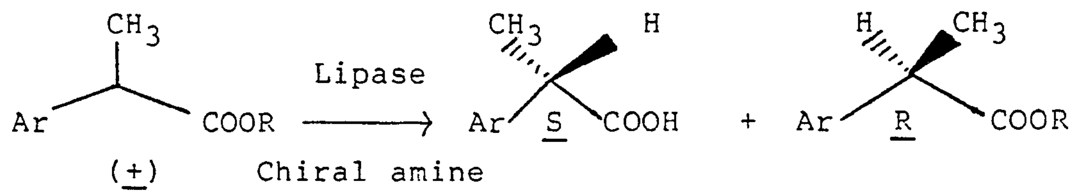


In the case of C. cylindracea lipase which has S-stereochemical preference for this class of substrates, the enantioselectivity commonly expressed as the E value is markedly enhanced in the presence of the chiral amine by preferentially inhibiting the hydrolysis of the R-ester. Typically

R = $CH_2CH_2$ Cl, C≡N, alkyl (1-4 carbons)

Ar = aryl or substituted aryl or condensed with other aromatic rings

In the case of (±)-2-aroylpropionic esters, the C. cylindracea lipase has an R-stereochemical preference for this class of substrates and in the presence of a chiral amine, the kinetic resolution becomes more enantioselective because the chiral amine preferentially inhibits the hydrolysis of the S-ester.

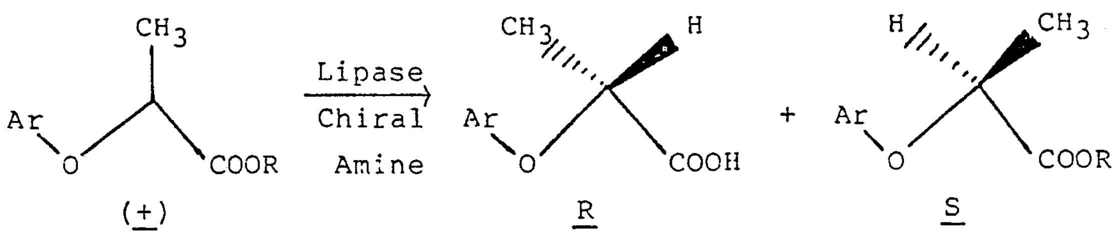


Ar = aryl and halogenated aryl or condensed with other aromatic rings

R = $CH_2CH_2Cl$, C≡N, alkyl (1-4 carbons)

While a variety of chiral amines can be employed such as dextromethorphan, levomethorphan, quinidine, brucine, morphine, quinine, cinchonidine, cinchonine, arylamines, phenylglycinol, ephedrine, alpha-methylbenzylamine, naphthylethylamine, phenylethylamine, and strychnine, the choice of the specific amine depends on the particular substrate structure and appropriate concentration must be defined. For example, normally a suitable concentration of the chiral amine is in the range of 0.001M to 0.1M with 0.01M to 0.03M to be optimum in many cases. The esters of (±)-2-arylpropionic and (±)-2-aroylpropionic acids which are to be resolved can be prepared by conventional methods (see, I. T. Harrison and S. Harrison in Compendium of Organic Synthetic Methods, Chapter 8, John Wiley & Sons, N.Y., 1971, p. 271].

Although chiral amines are particularly suitable for use as the inhibitor in the process of the present invention, other inhibitors include: amino acids and derivatives thereof: alkaloids; peptides; heterocycles; and aminosugars. The choice of the specific inhibitor to be employed depends largely upon the particular racemic substrate to be resolved.

The biocatalyst which may be used according to the process of this invention may be an enzyme such as a water-soluble lipase produced by microorganisms. The lipase of Candida cylindracea proved to be particularly suitable for the purpose of this invention. The extracellular microbial lipases are well known and many of these are available commercially [see M. Iwai and Y. Tsujsaka, p. 443, and M. Sugiura, p. 505, in Lipases, B. Borgstrom and H. L. Brockman (eds.), Elsevier, N.Y., 1984]. For example, they are used industrially for the transesterification of fats and were incorporated in laundry detergents for removal of oily contaminants. One outstanding feature of these microbial lipases that distinguishes them from intact microorganisms is that they can tolerate high substrate and product concentrations. For example, no marked substrate and product inhibition were noted. Hence, these enzymatic hydrolytic reactions can be carried out in high concentrations (0.1-5M) with an unusually high degree of enantioselectivity . Moreover, they are remarkably stable under the described reaction conditions, so that they may be reused.

Other biocatalysts that may be employed in the process of the present invention include the following: Procine pancreatic lipase; cholesterol esterase; pig liver esterase; lipases from the following organisms: Mucor miehei; Geotrichum candidum; Rhizopus delemar; Pseudomonas sp (AK Amano); Aspergillus niger; Chromobactenum violaceum; Subtilisin; Chymotrypsin; ficin; papain; Bromolain and microbial proteases. The choice of the specific biocatalyst to be employed depends on factors such as the particular racemic substrate to be resolved, its stability, and its tolerance of substrate or product concentrations, among others.

The racemic ester substrate may be added in solid or liquid forms at concentrations of, say, 0.1-5M to a suitable buffer solution containing the lipase to effect the enantioselective hydrolysis. Alternatively, the substrate can be dissolved in a suitable organic solvent such as carbon tetrachloride, cyclohexane, carbon disulfide, or hexane, as long as the solvent does not denature the enzyme. In addition, the substrate may be emulsified by the use of polyvinyl alcohol or propylene glycol. Of course, the temperature and pressure conditions under which the contact of the racemic ester substrate with the lipase are interdependent as will be apparent to those skilled in the art. Generally, at atmospheric pressure, the temperature can range from about 10°C to about 60°C, but preferably between 20°C to 40°C, and the pH of the medium can range from 3 to about 9, but preferably between 6-8.5.

The duration of the enantioselective hydrolysis reaction may vary from several hours to several days, depending upon the specific activity of the enzyme used.

At the end of the reaction, the optically-active acid and the ester may be extracted from the reaction mixture by using water immiscible organic solvents such as ethyl acetate, methylene dichloride, diethyl ether, etc. From the concentrated organic extract, the acid may bc separated from the ester by column chromatographic methods using silica gel as support. Alternatively, the acid may be separated from ester by differential extraction [Q. M. Gu et al, Tet. Lett., 27 (1986) 1763].

## 5. General procedure for the biocatalytic enantioselective hydrolysis of (+)-substituted chiral propionates

A. No inhibitor -- Microbial lipase (30 mg) was suspended in 3 ml of 0.2M phosphate buffer, pH 8.0 or as indicated in Table 1. To the suspension was added 0.9 mmol of esters of (±)-arylpropionate or (±)-aroylpropionate. The reaction mixture was stirred at 24°C and the progress of the reaction was monitored by TLC or HPLC. When the conversion reached 30% to 50%, the mixture was terminated by acidification to pH 2.0 with aqueous 1N HCl and then extracted with ethyl acetate (3x4 ml). The organic phase was dried over anhydrous $Na_2SO_4$ and concentrated to dryness under reduced pressure. The crude residue was chromatographed over a silica gel column (0.8x12 cm, silica gel for flash chromatography, average 40 µm, J. T. Baker Chemical Co.). The column was eluted with a solvent system consisting of hexane-ethyl acetate-acetic acid (7:1:0.1). The remaining substrate ester was directly employed to determine the enantiomeric excess ($ee_S$) and the product acid was methylated by diazomethane treatment and the enantiomeric excess ($ee_P$) was determined by PMR analysis in the presence of the chiral shift reagent Tris[3-(heptafluoropropylhydroxymethylene)-(+)-camphorato]europium (III), Eu(hfc)$_3$. In the presence of Eu(hfc)$_3$ - (200 MHz PMR, $CDCl_3$, about 0.5 molar ratio of Eu(hfc)$_3$ to the ester), the doublet of $\alpha$-$CH_3$ was split into two peaks. The singlet of $-CO_2CH_3$ was also split into two peaks. The enantiomeric excess (ee) was calculated from the ratio of the areas of the two enantiomers based either on the signal of the $\alpha$-$CH_3$ group in most cases or the $-CO_2CH_3$ group for methyl-2-(4-chlorophenoxy)propionate.

B. With inhibitor -- The procedure was the same as A except the buffer containing an inhibitor was employed. For this purpose, 0.09 mmol of an inhibitor (chiral amine) was suspended in 2.5 ml of 0.2M phosphate buffer. The pH was adjusted to pH 8.0 or as indicated in the table with 0.2M $NaH_2PO_4$ and the total volume was then adjusted to 3 ml with 0.2M phosphate buffer.

The following examples are presented to illustrate the concept of this invention and are not to be considered as limiting in any way the scope of the appended claims. The concept of this invention is applicable to all biocatalytic kinetic resolution procedures.

## 6. Examples

### EXAMPLE 1

Chloroethyl-2-phenylpropionate.

Dextromethorphan-free base (24.4 mg, 0.9 mmol) was suspended in 2.5 ml of 0.2M, pH 8.0 phosphate buffer. The pH of the suspension was adjusted to 8.0 with 0.2M $NaH_2PO_4$ (about 0.3 ml) and the total volume was adjusted to 3 ml with pH 8.0 phosphate buffer. Lipase powder (30 mg) (Candida cylindracea , Sigma) was then added to the above buffer and stirred for 1 minute. To this suspension was added 191 mg (0.9 mmol) of (±)-chloroethyl-2-phenylpropionate and the reaction mixture was stirred (magnetic stirrer) at 24° C for 120 h. The reaction was then terminated by acidification to pH 2.0 using 1N HCl and the mixture was then extracted with ethyl acetate (3x4 ml). The organic extract was dried over anhydrous $Na_2SO_4$ and evaporated to dryness under reduced pressure. The crude residue was chromatographed over silica gel (average particle diameter 40 µm, J. T. Baker Chemical Co.) column (0.8x12 cm) and the column was eluted with hexane-ethyl acetate-acetic acid (7:1:0.1). Fractions 3 to 5 (5 ml/fraction) were combined to yield the pure remaining substrate, which was analyzed using the PMR method in the presence of the chiral shift reagent Tris[3-(heptafluoropropylhydroxymethylene)-(+)-camphorato]europium (III), $Eu(hfc)_3$ (200 MHz PMR, $CDCl_3$, 0.4 molar ratio of $Eu(hfc)_3$ to the ester). The doublet of $\alpha$-$CH_3$ was split into two peaks and the peaks of the (S)-(+)-enantiomer were more downfield than those of the (R)-(-)-enantiomer. The ee was calculated by the ratio of the integration areas of the signals of the $\alpha$-$CH_3$ group. The enantiomeric excess of the remaining substrate ($ee_S$) was found to be 0.39. Fractions 10 to 14 were combined to give the pure product acid, which was converted into its methyl ester by treatment with diazomethane. Its enantiomeric excess ($ee_P$) was determined similarly using the PMR method in the presence of $Eu(hfc)_3$. The results were as follows: $ee_S$ = 0.39, $ee_P$ = 0.98, c = 0.28, E > 100. The stereochemical preference was found to be the (S)-(+)-enantiomer.

A similar experiment was conducted without inhibitor using the same experimental conditions except in this case 3 ml of 0.2M phosphate buffer (pH 8.0) was used. The results of this control sample were: $ee_S$ = 0.80, $ee_P$ = 0.62, c = 0.56, E = 10 and the same (S)-(+)-stereochemical preference was observed. This experiment demonstrates that dextromethorphan preferentially inhibited the hydrolysis of (R)-(-)-chloroethyl-2-phenylpropionate by the lipase of Candida cylindracea and thereby enhanced the enantioselectivity (E) of the reaction more than 10 fold. The results of the analyses are shown in Figure 1.

EXAMPLE 2

Methyl 2-(2,4-dichlorophenoxy)propionate.

Methyl 2-(2,4-dichlorophenoxy)propionate was hydrolyzed in phosphate buffer catalyzed by lipase of Candida cylindracea with no enantioselectivity (E = 1).

Dextromethorphan-free base (24.4 mg, 0.09 mmol) was suspended in 2.5 ml of 0.2M, pH 8.0 phosphate buffer, and the pH of the suspension was adjusted to 8.0 with 0.2M $NaH_2PO_4$ (0.3 ml); the total volume of the mixture was adjusted to 3 ml with pH 8.0 phosphate buffer. Lipase powder (30 mg) (Candida cylindracea, Sigma) was added to the above buffer and stirred (magnetic stirrer) for 1 minute. To the suspension was then added 224 mg (0.9 mmol) of (±)-methyl-2-(2,4-dichlorophenoxy)propionate. The mixture was stirred (magnetic stirrer) at 24° C for 42 hours and the progress of the reaction was monitored by TLC (hexane-ethyl acetate-acetic acid, 5:1:0.05). When the conversion reached 30% to 50%, the reaction mixture was acidified with aqueous 1N HCl to pH 2, and then extracted with ethylacetate (3x4 ml). The organic extract was dried over anhydrous $Na_2SO_4$ and evaporated to dryness under reduced pressure. The crude residue was chromatographed over a silica gel column (0.8x12 cm, silica gel for flash chromatography, average 40 µm, J. T. Baker Chemical Co.) and the column was eluted with hexane-ethyl acetate-acetic acid (6:1:0.1). Fractions containing the pure remaining substrate ester were combined. The pure product acid was also combined and was then converted into its methyl ester by reaction with diazomethane. The enantiomeric excess of the remaining substrate ($ee_S$) and the product ($ee_P$) were again determined by the PMR method in the presence of the chiral shift reagent (0.8 molar ratio of $Eu(hfc)_3$ to the ester, in $CDCl_3$, 200 MHz PMR). The doublet of $\alpha$-$CH_3$ was split into two peaks and the peaks of the (R)-(+)-enantiomer were more downfield than those of the (S)-(-)-enantiomer. The ee was calculated from the ratios of the integral areas of the two enantiomers based on the signals of the $\alpha$-$CH_3$ group. The results

were as follows: $\underline{ee}_S$ = 0.38, $\underline{ee}_P$ = 0.87, c = 0.30, E = 20.

## EXAMPLES 3-9

Following the general procedures described above and with reactants indicated, the optical purities expressed as enantiomeric excess ($\underline{ee}_S$ and $\underline{ee}_P$) are shown in Table 1.

TABLE 1

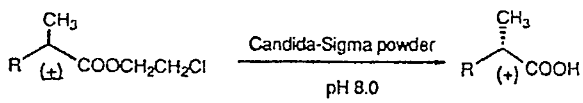


| Example | R | Inhibitor | Reaction time (h) | $ee_S$ | $ee_P$ | c | E | Stereochemical preference |
|---|---|---|---|---|---|---|---|---|
| | 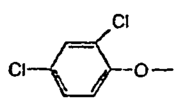 | None | 10 | 0 | 0 | 0.34* | 1 | |
| 3 | | Quinidine 0.03M | 10 | 0.15 | 0.61 | 0.20 | 5 | (R)-(+)- |
| 4 | | Quinine 0.03M | 18 | 0.15 | 0.52 | 0.22 | 4 | (R)-(+)- |
| 5 | | Cinchonidine 0.03M | 18 | 0.16 | 0.34 | 0.32 | 2.4 | (R)-(+)- |
| 6 | | Brucine 0.03M | 18 | 0.14 | 0.36 | 0.28 | 2.4 | (R)-(+)- |
| 7 | 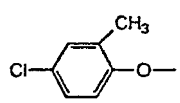 | None | 22 | 0 | 0 | 0.5* | 1 | |
| | | Dextromethorphan 0.03M | 30 | 0.31 | 0.93 | 0.25 | 37 | (R)-(+)- |
| 8 | 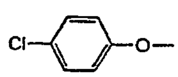 | None | 0.7 | 0.26 | 0.81 | 0.24 | 12 | (R)-(+)- |
| | | Dextromethorphan 0.07M | 0.7 | 0.10 | 0.96 | 0.09 | 54 | (R)-(+)- |
| 9 | 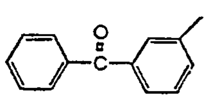 | None | 120 | 0.25 | 0.52 | 0.32 | 4 | (S)-(+)- |
| | | Dextromethorphan 0.03M | 190 | 0.06 | 0.95 | 0.06 | 42 | (S)-(+)- |

*Determined by HPLC.

## Claims

1. A method of improving the enantioselectivity of biocatalytic resolution of racemic compounds, which comprises reacting enantiomers of the racemic compound with a biocatalyst to form a reaction mixture in the presence of an inhibitor to said biocatalytic reaction process to selectively inhibit the reaction of one of

the enantiomers.

2. A method according to claim 1 wherein the enantiomers of said racemic compound include a fast-reacting enantiomer and a slow-reacting enantiomer, and said inhibitor inhibits the reaction of said slow-reacting enantiomer.

3. A method according to claim 1 or 2 wherein said reaction mixture includes an optically active compound.

4. A method according to claim 3 wherein said optically active compound is a chiral non-racemic hydroxy, amino, halo, epoxy or alkyl, including carboxylic, acid or a derivative thereof.

5. A method according to claim 3 wherein said optically active compound is an arylpropionic acid or aroylpropionic acid.

6. A method according to any one of claims 1 to 5 wherein said biocatalytic reaction process is a hydrolytic reaction.

7. A method according to claim 6 wherein said biocatalyst is a hydrolase.

8. A method according to claim 7 wherein said hydrolase is an extracellular microbial lipase.

9. A method according to any one of claims 1 to 6 wherein said biocatalyst is Candida cylindracea lipase, porcine pancreatic lipase, cholesterol esterase; pig liver esterase; or a lipase from the following organisms: Mucor miehei; Geotrichum candidum; Rhizopus delemar; Pseudomonas sp (AK Amano); Aspergillus niger; Chromobactenum violaceum; Substilisin; Chymotrypsin; ficin; papain, Bromolain or a microbial protease.

10. A method according to any one of claims 1 to 9 wherein the inhibitor is a partial non-competitive enantioselective inhibitor.

11. A method according to any one of claims 1 to 10 wherein the inhibitor is a chiral amine, amino acid, or a derivative thereof, an alkaloid, peptide, heterocycle or aminosugar.

12. A method according to claim 11 wherein the inhibitor is a chiral amine.

13. A method according to claim 12 wherein said chiral amine is dextromethorphan, levomethorphan, quinidine, quinine, cinchonine, strychnine, cinchonidine, brucine, morphine, naphthylethlamine, phenylethylamine, phenylglycinol, ephedrine, or alpha-methylbenzylamine.

14. A method according to any one of claims 1 to 13 wherein said racemic compound is a chiral racemic ester which is an ester of an amino, hydroxy, or halo carboxylic acid or a derivative thereof.

15. A method according to any one of the preceding claims which comprises reacting enantiomers of a racemic ester with a biocatalyst and an inhibitor which selectively inhibits one of the enantiomer reactions, to form a reaction mixture containing an optically active compound and the said inhibited enantiomer; and recovering the optically active compound from the reaction mixture.

16. A method according to claim 15 wherein the step of recovering comprises extracting the optically active compound and inhibited enantiomer with a water immiscible organic solvent to provide a concentrated organic extract, and thereafter separating the optically active compound from the extract.

FIGURE 1

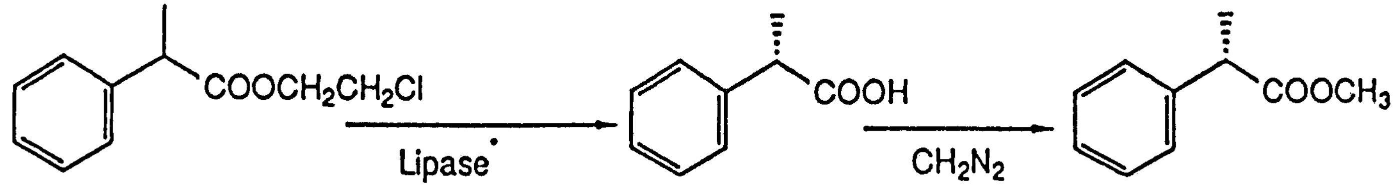


(±)

(S)-(+)-

No Inhibitor

With 0.03M Dextromethorphan

Remaining Sub.

Product

Remaining Sub.

Product

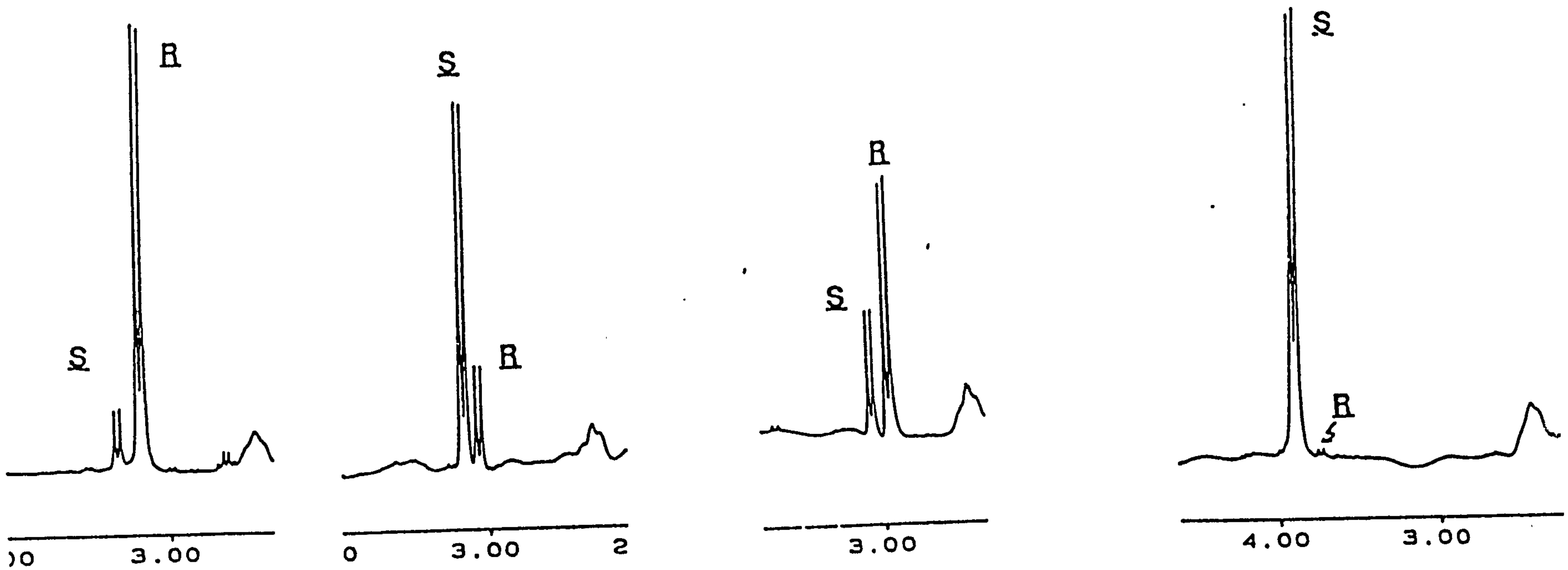


$ee_S = 0.80$

$ee_P = 0.62$

$ee_S = 0.39$

$ee_P = 0.98$

c = 0.56
E = 10

c = 0.28
E > 100

*Crude powder of *Candida cylindracea* (Sigma), 24°C.

European Patent Office

# EUROPEAN SEARCH REPORT

Application number

EP 90302497.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | EP - A2 - 0 290 878 (CHISSO CORPORATION) * Claims 5,6 * -- | 1,6-9, 15 | C 12 P 41/00 |
| A | FR - A1 - 2 603 304 (INSTITUT DE RECHERCHES CHIMIQUES ET BIOLOGIQUES APPLIQUEES I.R.C.E.B.A.) * Claim 1 * -- | 1,6-9 | |
| A | WO - A1 - 87/05 328 (WISCONSIN ALUMNI RESEARCH FOUNDATION) * Claims 1,6-10,12-15 * -- | 1,6-9 | |
| A | EP - A2 - 0 303 947 (HOECHST AG) * Claims 1,2 * -- | 1,6-9 | |
| A | DD - A1 - 258 625 (MASSACHUSETTS INSTITUTE OF TECHNOLOGY) * Claims 1,4 * ---- | 1,6-9 | TECHNICAL FIELDS SEARCHED (Int Cl.5) |
| | | | C 12 P |

The present search report has been drawn up for all claims

| Place of search | Date of completion of the search | Examiner |
|---|---|---|
| VIENNA | 11-05-1990 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document